# EUROPEAN PATENT SPECIFICATION

(11) **EP 3 747 478 B1**
(45) Date of publication and mention of the grant of the patent: **12.07.2023**
(21) Application number: 18903984.5
(22) Date of filing: 19.10.2018
(51) Int. Cl.: A61L 27/60, A61L 27/36, C12N 5/0775, B33Y 80/00, B33Y 70/00, A61L 27/22, A61L 27/38

(54) **BIOINK COMPOSITION FOR DERMIS REGENERATION SHEET, METHOD FOR MANUFACTURING CUSTOMIZED DERMIS REGENERATION SHEET USING SAME, AND CUSTOMIZED DERMIS REGENERATION SHEET MANUFACTURED USING MANUFACTURING METHOD**
BIOTINTENZUSAMMENSETZUNG FÜR HAUTREGENERATIONSFOLIE, VERFAHREN ZUR HERSTELLUNG EINER PERSONALISIERTEN HAUTREGENERATIONSFOLIE DAMIT UND NACH DEM HERSTELLUNGSVERFAHREN HERGESTELLTE PERSONALISIERTE HAUTREGENERATIONSFOLIE
COMPOSITION D'ENCRE BIOLOGIQUE POUR FEUILLE DE RÉGÉNÉRATION DU DERME, PROCÉDÉ DE FABRICATION DE FEUILLE DE RÉGÉNÉRATION DU DERME PERSONNALISÉE FAISANT APPEL À CELLE-CI, ET FEUILLE DE RÉGÉNÉRATION DU DERME PERSONNALISÉE FABRIQUÉE EN FAISANT APPEL AU PROCÉDÉ DE FABRICATION

(30) Priority: 31.01.2018 KR 20180012220
(43) Date of publication of application: 09.12.2020
(73) Proprietor: Rokit Healthcare Inc., Geumcheon-gu Seoul 08512 (KR)
(72) Inventor: YOU, Seok Hwan, Seoul 06943 (KR)
(74) Representative: Maiwald GmbH
(86) International application number: PCT/KR2018/012430
(87) International publication number: WO 2019/151611

(56) References cited:
- EP-A1- 2 374 485
- KR-A- 20140 084 013
- KR-A- 20150 020 702
- KR-A- 20160 115 208
- KR-A- 20170 012 099
- US-A1- 2003 175 410
- HE PENG ET AL: "Abstract", BURNS & TRAUMA, [Online] vol. 6, 23 January 2018 (2018-01-23), XP055815247, DOI: 10.1186/s41038-017-0104-x Retrieved from the Internet: URL:http://academic.oup.com/burnstrauma/ar ticle-pdf/doi/10.1186/s41038-017-0104-x/36 112302/burns_v6_1_104.pdf>
- KLAR AGNIESZKA S ET AL: "Tissue-engineered dermo-epidermal skin grafts prevascularized with adipose-derived cells", BIOMATERIALS, ELSEVIER, AMSTERDAM, NL, vol. 35, no. 19, 27 March 2014 (2014-03-27), pages 5065-5078, XP028847267, ISSN: 0142-9612, DOI: 10.1016/J.BIOMATERIALS.2014.02.049
- KATHARINA WITTMANN ET AL: "Engineering Vascularized Adipose Tissue Using the Stromal-Vascular Fraction and Fibrin Hydrogels", TISSUE ENGINEERING PART A, vol. 21, no. 7-8, 1 April 2015 (2015-04-01), pages 1343-1353, XP055746766, US ISSN: 1937-3341, DOI: 10.1089/ten.tea.2014.0299

## Description

### Technical Field

The present specification claims priority to and the benefit of Korean Patent Application No. 10-2018-0012220 filed in the Korean Intellectual Property Office on January 31, 2018.

The present invention relates to a bioink composition for a dermis regeneration sheet, a method for manufacturing a customized dermis regeneration sheet using the same, and a customized dermis regeneration sheet manufactured using said manufacturing method.

### Background Art

The skin is the largest organ which covers the entire surface of the human body, and functions to prevent the loss of body fluids, prevent the inflow of harmful materials and microorganisms from the outside, and protect our bodies from physical irritation, radiation, ultraviolet rays, and the like. The skin has various accessory organs such as hair follicles, hair, sweat glands, and sebaceous glands, and thus is an important complex organ which performs various functions in addition to the protective film function. The skin is roughly divided into the epidermis, the dermis, and the subcutaneous tissue (hypodermis).

The dermis is a layer below the epidermis, is a layer which supplies a substrate which supports various structures such as blood vessels and nerves, and consists of collagen, elastic fibers, and extracellular matrices. The dermis largely consists of a papillary layer, in which fibroblasts are abundant in the upper region and microvessels are distributed and a reticular connective tissue in which collagen fibers are abundant.

A part of the skin tissue may be damaged by burns, trauma, skin diseases, and in this case, an autograft method for implanting the skin tissue of the person for the purpose of healing damaged tissue or for reconstructive surgery, a homograft or allograft method for implanting the skin of another person, and a heterograft or xenograft method for implanting the skin of an animal are used. Among these methods, the autograft is the most ideal, but has disadvantages in that when a treatment site is wide, the site where the tissue can be secured is limited, and the harvest site remains as a new wound site. The homograft or allograft serves to aid in the migration and healing of cells around a wound.

Artificial skin is three-dimensionally reconstructed skin using skin cells and skin components such as collagen and elastin, and is called a skin equivalent or reconstructed skin because the artificial skin consists of living fibroblasts and keratinocytes to exhibit morphological and physiological characteristics similar to those of actual skin. Artificial skin was developed in the 1980s for the purpose of treating patients who require skin implantation due to severe burns, but their own skin cannot be implanted because the severity of the burn is too high or the affected area is too wide, and through continuous improvement and research, artificial skin is currently used in various fields such as skin physiology research, skin irritation evaluation, and skin efficacy evaluation. However, existing artificial skin has a problem that a phenomenon in which the dermis gradually contracts during tissue culture in the manufacturing method occurs, and when the dermis contracts sharply, the artificial skin is entirely deformed.

In the case of existing cell therapeutic agents, a method for implanting allogeneic cells using the allogeneic cells or a method of transplanting autologous cells has been used. The implantation of allogeneic cells has a paracrine effect, but has a problem in that the body views the allogeneic cells as a foreign body and the allogeneic cells eventually disappear. Further, when autologous cells are implanted by an injection method, it is highly likely that the cells will disappear when there is no environment (for example, a scaffold) for the cells to grow, and eventually, it is difficult to see an effect caused by injected cells.

In addition, the autologous implantation in which a part of an autologous tissue is isolated, cultured, and proliferated has problems such as the possibility of scarring of a donor site and the lack of a donor site when a site to be implanted is wide, and the allograft and the xenograft have problems such as immune rejection responses or induction of inflammation. Therefore, there is a need for developing a method capable of replacing or regenerating damaged skin and minimizing the side effects as described above.

### Prior Art Documents

He P, Zhao J, Zhang J, Li B, Gou Z, Gou M, Li X. Bioprinting of skin constructs for wound healing. Burns Trauma. 2018 Jan 23;6:5.

### Patent

### Document

Korean Patent No.: 10-1710615

### Disclosure

### Technical Problem

The present invention has been made in an effort to provide a bioink composition for a dermis regeneration sheet capable of minimizing an implantation rejection response and manufacturing a patient customized dermis regeneration sheet, a method for manufacturing a customized dermis regeneration sheet using the same, and a dermis regeneration sheet.

### Technical Solution

The scope of this invention is defined by the claims. Embodiments in the description relating to methods of treatment are not covered by the claims. Any "embodiment" or "example" which is disclosed in the description but is not covered by the claims should be considered as presented for illustrative purpose only.

An exemplary embodiment of the present invention provides a bioink composition for a dermis regeneration sheet, the bioink composition comprising: a first liquid comprising an adipose tissue-derived stromal vascular fraction, an adipose tissue-derived extracellular matrix, and fibrinogen, wherein a content of the adipose tissue-derived extracellular matrix in the first liquid is 20 wt% to 60 wt%; and a second liquid comprising thrombin, wherein a concentration of thrombin in the second liquid is 40 IU/ml to 250 IU/ml.

Another exemplary embodiment of the present invention provides a method for manufacturing a customized dermis regeneration sheet, the method comprising:
a) obtaining 3D data of a defect dermis site using a 3D scanner, a1) manufacturing a mold corresponding to a shape of the obtained 3D data using a 3D printer,
b) forming a first layer corresponding to a shape of the obtained 3D data using a first liquid comprising an adipose tissue-derived stromal vascular fraction, an adipose tissue-derived extracellular matrix, and
   fibrinogen, wherein a content of the adipose tissue-derived extracellular matrix in the first liquid is 20 wt% to 60 wt%, and wherein in step b), the first liquid is applied in the mold;
c) forming a second layer by applying a second liquid comprising thrombin onto the first layer, wherein a concentration of thrombin in the second liquid is 40 IU/ml to 250 IU/ml;
and d) forming a dermis regeneration sheet by allowing the first layer and the second layer to react with each other.

Still another exemplary embodiment of the present invention provides a customized dermis regeneration sheet manufactured using the manufacturing method.

### Advantageous Effects

A bioink composition for a dermis regeneration sheet according to the present invention and a method for manufacturing a customized dermis regeneration sheet using the same have an advantage in that a dermal regeneration sheet suitable for a damaged skin tissue of a patient can be manufactured. Specifically, the method for manufacturing a customized dermis regeneration sheet according to the present invention can manufacture a dermis regeneration sheet having a shape matching an affected area by 3D-scanning an accurate shape of the affected area.

The dermis regeneration sheet according to the present invention has an advantage in that implantation can be performed without an immune rejection response, and also has an advantage in that healthy cells can be implanted into an affected area because the dermis regeneration sheet can be manufactured within a short period of time.

### Description of Drawings

FIG 1 illustrates a process of respectively preparing a first liquid and a second liquid in order to manufacture a customized dermis regeneration sheet according to the present invention.
FIG 2 shows photographs confirming the cell viability of the customized dermis regeneration sheet according to Experimental Example 1.
FIG 3 shows a photograph in which the skin is removed in an animal experiment for Experimental Example 2.
FIG 4 shows a control prepared according to Experimental Example 2 and a customized dermis regeneration sheet of the Example.
FIG 5 shows the progress of the implanted customized dermis regeneration sheets according to Experimental Example 2.
FIG 6 shows the results of performing Masson's Trichrome staining in an animal experiment of Experimental Example 2.
FIG 7 shows the presence or absence of angiogenesis at an implanted site as a result of the animal experiment according to Experimental Example 2 using CD31 staining.

### Modes of the Invention

When one member is disposed "on" another member in the present specification, this includes not only a case where the one member is brought into contact with another member, but also a case where still another member is present between the two members.

When one part "includes" one constituent element in the present specification, unless otherwise specifically described, this does not mean that another constituent element is excluded, but means that another constituent element may be further included.

Hereinafter, the present invention will be described in detail.

An exemplary embodiment of the present invention provides a bioink composition for a dermis regeneration sheet, the bioink composition comprising: a first liquid comprising an adipose tissue-derived stromal vascular fraction, an adipose tissue-derived extracellular matrix, and fibrinogen; and a second liquid comprising thrombin.

The bioink composition for a dermis regeneration sheet according to the present invention is a two-liquid type, and the first liquid and the second liquid may be sequentially applied and then reacted to form a dermis regeneration sheet. Specifically, the thrombin in the second liquid may be reacted with the fibrinogen in the first liquid to form a fibrin network, which may serve to sufficiently fix the adipose tissue-derived stromal vascular fraction and the extracellular matrix.

The adipose tissue-derived stromal vascular fraction includes adipose derived stem cells. Preferably, the adipose tissue-derived stromal vascular fraction may not substantially include cells (for example, adipocytes, erythrocytes, other stromal cells, and the like) other than adipose tissue-derived stem cells and extracellular matrix materials, and more preferably, may not include other cells and extracellular matrix materials at all.

The adipose tissue-derived stromal vascular fraction may be extracted from an adipose tissue of an allogeneic animal or a heterologous animal. Preferably, the adipose tissue-derived stromal vascular fraction may be extracted from an autologous adipose tissue. More specifically, the adipose tissue-derived stromal vascular fraction may be extracted using an adipose tissue of a patient or animal to be treated. The adipose tissue-derived stromal vascular fraction may be obtained by extracting the adipose tissue-derived stromal vascular fraction (SVF), an extracellular matrix (ECM), and the like in the form of a micro or nano SVF/ECM cluster from an adipose tissue, and a pure adipose tissue-derived stromal vascular fraction (SVF) and extracellular matrix (ECM) may also be separated from each other and used. As an example, the adipose tissue-derived stromal vascular fraction may be extracted from an adipose tissue using a Lipocell kit from Tiss'you. However, the present invention is not limited thereto, and the adipose tissue-derived stromal vascular fraction may be obtained using a method, an extraction kit, and the like known in the art.

According to an exemplary embodiment of the present invention, a concentration of the adipose tissue-derived stromal vascular fraction in the first liquid may be 10⁵ cells/ml to 10⁷ cells/ml. When the concentration of the adipose tissue-derived stromal vascular fraction is within the above-described range, a customized dermis regeneration sheet to be manufactured can effectively differentiate the adipose tissue-derived stromal vascular fraction into dermal cells at an implantation site.

The higher the content of the adipose tissue-derived stromal vascular fraction is, the more the effect is improved, but when the content thereof is extremely high, it may be difficult for the effect caused by the extracellular matrix, fibrinogen, and the like to be expressed. Therefore, the content of the adipose tissue-derived stromal vascular fraction may balance the contents of the other constituent components in the first liquid.

The adipose tissue-derived stromal vascular fraction is used with an adipose tissue-derived extracellular matrix to promote differentiation into dermal cells. Specifically, the adipose tissue-derived extracellular matrix has biochemical factors necessary for the growth and differentiation of cells essential for wound healing in an affected area, and may provide a physical environment in which the adipose tissue-derived stromal vascular fraction may differentiate into dermal cells and then fixed.

The extracellular matrix is extracted from an adipose tissue which may be of an allogeneic or heterologous animal.

Preferably, the extracellular matrix is extracted from an autologous adipose tissue.

According to an exemplary embodiment of the present invention, the extracellular matrix may be decellularized.

According to an exemplary embodiment of the present invention, the extracellular matrix may be particles having a diameter of 5 µm to 100 µm. Specifically, the extracellular matrix may be decellularized and pulverized by a physical method and prepared in a particulate form. Furthermore, the extracellular matrix may be obtained using methods known in the art.

A fibrin matrix through a reaction of fibrinogen of the first liquid and thrombin of the second liquid may serve to fix the adipose tissue-derived stromal blood vascular fraction and the extracellular matrix.

According to the claimed invention, a content of the extracellular matrix in the first liquid is 20 wt% to 60 wt%. Preferably, the content of the extracellular matrix in the first liquid is 20 wt% to 50 wt%.

When the content of the extracellular matrix is within the above range, the viability of cells differentiating into dermal cells may be improved to effectively achieve the regeneration of a defect dermis site. When the content of the extracellular matrix is too high, the contents of fibrinogen and thrombin for manufacturing a dermis regeneration sheet are relatively decreased, so that there may be a problem in that it is difficult to maintain the form of a prepared dermis regeneration sheet. Further, when the content of the extracellular matrix is too low, the contents of fibrinogen and thrombin for manufacturing a dermis regeneration sheet are extremely increased, and as a result, the manufactured dermis regeneration sheet becomes extremely hard, so that there may be a problem in that it is difficult to expect an effect caused by the extracellular matrix. Therefore, it is preferred to improve the viability of cells differentiating into dermal cells by adjusting the content of the extracellular matrix to the above range, and furthermore, to facilitate the maintenance of the shape of the dermis regeneration sheet.

According to an exemplary embodiment of the present invention, a concentration of fibrinogen in the first liquid may be 4 mg/ml to 50 mg/ml. In addition, the concentration of fibrinogen in the first liquid may be 5 mg/ml to 40 mg/ml, or 7 mg/ml to 30 mg/ml.

According to the present invention, the concentration of thrombin in the second liquid is 40 IU/ml to 250 IU/ml, preferably 40 IU/ml to 100 IU/ml, or 45 IU/ml to 80 IU/ml.

When the concentration of fibrinogen and thrombin are within the above ranges, the curing rate may be appropriately secured and the distribution of the adipose tissue-derived stromal vascular fraction may be evenly maintained. Through this, the distribution of cells may be uniformly maintained in a customized dermis regeneration sheet to be manufactured, and cell differentiation ability after implantation of the customized dermis regeneration sheet may be effectively performed. In addition, when the concentrations of fibrinogen and fibrinogen ae within the above ranges, there are advantages in that the form of the manufactured dermis regeneration sheet may be maintained well, and the sheet may be suitably implanted into an affected area by maintaining an appropriate hardness.

According to an exemplary embodiment of the present invention, the first liquid may further include aprotinin. The aprotinin is an inhibitor of proteolytic enzymes secreted from the pancreas, and is a polypeptide consisting of a total of 58 amino acids. The aprotinin is usually extracted from the lungs of cattle, and is known to perform a hemostatic action by inhibiting the degradation of fibrin in blood.

According to an exemplary embodiment of the present invention, the aprotinin may be included in an amount of 900 kininogen inactivator units (KIU) to 1,1000 KIU, specifically 1,000 KIU, per ml of the first liquid.

According to an exemplary embodiment of the present invention, the second liquid may be thrombin dispersed in a calcium chloride solution. Specifically, the second liquid includes 40 IU to 250 IU of thrombin and 5 mg to 6.5 mg of calcium chloride per ml of the second liquid.

A solvent of the first liquid and the second liquid may be water, specifically physiological saline. Further, the fibrinogen in the first liquid and the thrombin in the second liquid may be obtained by a commercially available fibrin glue kit.

Since the reaction of the first liquid and the second liquid is completed within 10 minutes, preferably within 5 minutes, the bioink composition for a dermis regeneration sheet has an advantage in that a dermis regeneration sheet may be immediately manufactured using a 3D printer at a treatment site. This is a reference-embodiment which is not part of the claimed invention.

The present invention uses a fibrin glue including fibrinogen and thrombin as an adhesive, which may secure higher viscosity than that of a hyaluronic acid adhesive or collagen adhesive, so that the customized dermis regeneration sheet has excellent bonding force to an affected area, and furthermore, may maintain high strength.

The present invention provides a method for manufacturing a customized dermis regeneration sheet, the method comprising:
a) obtaining 3D data of a defect dermis site using a 3D scanner; a1) manufacturing a mold corresponding to a shape of the obtained 3D data using a 3D printer;
b) forming a first layer corresponding to a shape of the obtained 3D data using a first liquid comprising an adipose tissue-derived stromal vascular fraction, an adipose tissue-derived extracellular matrix, and
   fibrinogen, wherein a content of the adipose tissue-derived extracellular matrix in the first liquid is 20 wt% to 60 wt%, and wherein in step b), the first liquid is applied in the mold;
c) forming a second layer by applying a second liquid comprising thrombin onto the first layer, wherein a concentration of thrombin in the second liquid is 40 IU/ml to 250 IU/ml;
and d) forming a dermis regeneration sheet by allowing the first layer and the second layer to react with each other.

In step a), 3D scanner equipment or 3D printing equipment known in the art may be used. In addition, in step a1), 3D printing equipment known in the art may be used. The mold may serve to be able to fix a three-dimensional form when the first liquid and the second liquid are applied. The mold may be removed after the customized dermis regeneration sheet is formed. The mold may be formed using a biocompatible polymer generally used in the art.

According to an exemplary embodiment of the present invention, steps b) and c) may be performed using inkjet printing or 3D printing. Specifically, in steps b) and c), a printing device having two or more nozzles known in the art may be used, and a three-dimensional shape may be created by discharging the first liquid and the second liquid from the respective nozzles. Furthermore, when the first layer is formed, a uniform cell distribution may be obtained using a 3D printer or an inkjet printer, thereby preventing a cell aggregation phenomenon, and the like in the customized dermis regeneration sheet.

According to an exemplary embodiment of the present invention, steps b) and c) may be alternately repeated. Specifically, when it is necessary to form a large-volume dermis regeneration sheet, steps b) and c) are alternately performed to laminate layers as in "first layer/second layer/first layer/second layer, and then the layers may be coagulated to form a scaffold for cartilage regeneration.

According to an exemplary embodiment of the present invention, step d) may be completed within 10 minutes, preferably within 5 minutes. Specifically, in step d), a reaction may be performed for 3 minutes to 7 minutes and the first layer and the second layer may be reacted to form a customized dermis regeneration sheet.

In the method for manufacturing a customized dermis regeneration sheet according to the present invention, the adipose tissue-derived stromal vascular fraction and the adipose tissue-derived extracellular matrix may be extracted from an adipose tissue of a patient or animal and prepared without a separate culture step. After the first liquid is prepared using the same, the customized dermis regeneration sheet may be manufactured within a short period of time and implanted into an affected area, so that the activity of cells may be maximized.

Further, since a dermis regeneration sheet may be manufactured in the same manner as a shape of the affected area by the method for manufacturing a customized dermis regeneration sheet, a natural state after restoration may be realized by minimizing a gap between the implantation site and the dermis regeneration sheet.

Still another exemplary embodiment of the present invention provides a customized dermis regeneration sheet manufactured using the manufacturing method.

As described above, the customized dermis regeneration sheet may be implanted after being manufactured in a shape suitable for an affected area within a short period of time using an adipose tissue of a patient or an animal. It may be planned to restore damaged skin by implanting the dermis regeneration sheet into an affected area and protecting the affected area with a dressing. After the customized dermis regeneration sheet is implanted, an adipose tissue-derived stromal vascular fraction differentiates into dermal cells, the differentiated dermal cells may be fixed in the adipose tissue-derived extracellular matrix and the fibrin matrix to restore damaged skin.

Hereinafter, the present invention will be described in detail with reference to Examples for specifically describing the present invention. However, the Examples according to the present invention may be modified into various different forms, and it should not be interpreted that the scope of the present invention is limited to the Examples to be described below. The Examples of the present specification are provided for more completely explaining the present invention to those with ordinary skill in the art.

### Adipose tissue-derived stromal vascular fraction (SVF)

Adipose tissue-derived stromal vascular fraction (SVF) cells were obtained through the following steps.
1. Approximately 60 cc of adipose tissue was extracted by performing liposuction led by a doctor in a sterile operating room, and 0.075% collagenase was added in the same amount to the tissue, and the resulting mixture was allowed to react by shaking incubation at 230 rpm at a temperature of 37°C for 30 minutes.
2. After the reaction as described above, the reaction product was centrifuged at a speed of 420 g (relative centrifugal force (RCF)) at 25°C for 10 minutes, and as a result of the centrifugation, the reaction product was divided into three layers of an SVF pellet layer, a collagenase layer, and an oil layer.
3. After the upper solution except for the SVF pellet layer was removed and the SVF pellet layer was resuspended in phosphate buffered saline (PBS), fibrous materials and the remaining impurities of the resuspended SVF layer were removed using a 100 using a 100 µm nylon filter (cell strainer).
4. After a solution including the filtered SVF was resuspended and centrifuged three times, all remaining materials were removed except for the pellet at the bottom layer, and then as a result of calculating nucleated cells using a cell counter, it could be confirmed that 0.26 × 10⁶ to 2.2 × 10⁶ SVF cells were obtained.

### Extracellular matrix (ECM)

After an adipose tissue was extracted by performing liposuction led by a doctor's in a sterile operating room, the adipose tissue was mixed with physiological saline. Moreover, after the ECM was mechanically isolated from the adipose tissue using a homogenizer under the conditions of 12,000 rpm to 20,000 rpm, the isolated ECM was centrifuged. After a process of centrifuging the precipitate after centrifugation again was repeated 3 to 5 times, the ECM was finally obtained.

### Experimental Example 1 - In vitro experiment for confirming cell viability in customized dermis regeneration sheet

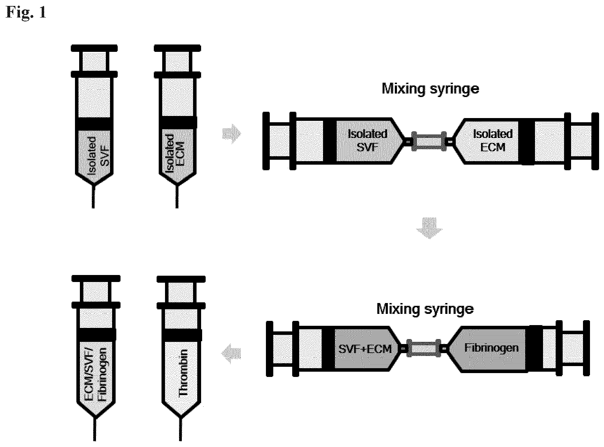

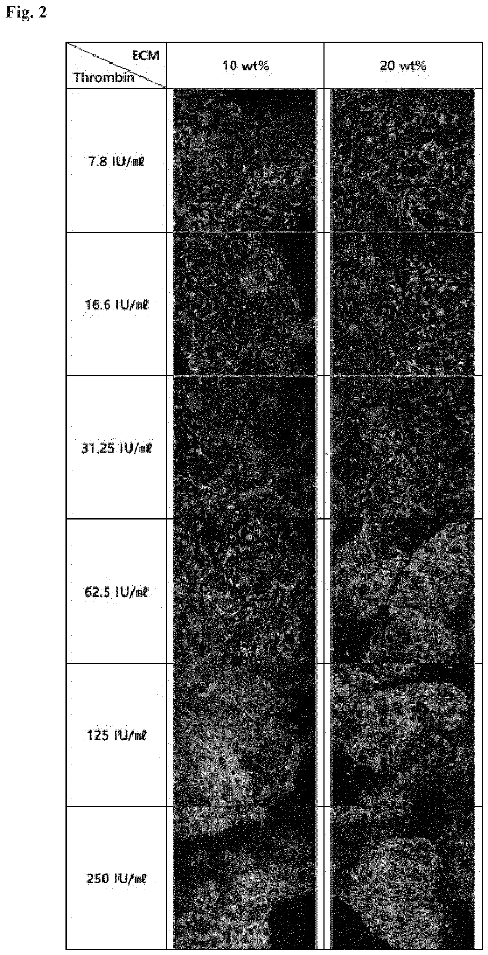

FIG 1 illustrates a process of respectively preparing a first liquid and a second liquid in order to manufacture a customized dermis regeneration sheet according to the present invention. Specifically, after the obtained SVF and ECM were mixed using a mix syringe, a first liquid was prepared by mixing 1 ml of an aprotinin liquid in which 4.5 mg/ml fibrinogen was mixed with the SVF/ECM mixture using a mix syringe. In this case, the content of the ECM in the first liquid was adjusted to 10 wt% (reference-example, not according to the claimed invention) and to 20 wt% (example according to the claimed invention) as shown in FIG 2.

Furthermore, a calcium chloride solution in which thrombin having the same volume as the prepared first liquid was dispersed was prepared. In this case, the concentration of thrombin in the second liquid was adjusted to 7.8 IU/ml to 250 IU/ml as shown in FIG 2. Hereby only the embodiments comprising from 40 IU/ml to 250 IU/ml thrombin are according to the claimed invention. The other examples are to be considered as reference-examples.

After a first layer was formed by applying the prepared first liquid, a customized dermis regeneration sheet was manufactured by applying the prepared second liquid onto the first layer.

FIG 2 shows photographs confirming the cell viability of the customized dermis regeneration sheet according to Experimental Example 1. Specifically, FIG 2 confirms the cell viability using a confocal microscope (Leica, TCS SP5) after green fluorescence calcein-AM staining for confirming living cells and red fluorescence-ethidium homodimer-1 staining for confirming whether the plasma membrane was lost were performed on the customized dermis regeneration sheet. According to FIG 2, when the content of the ECM was 20 wt%, the interaction between SVF and ECM was improved, so that the proliferation of cells was activated, and furthermore, when the concentration of thrombin was 50 IU/ml or more, it can be confirmed that cell proliferation is further activated.

### Experimental Example 2 - Animal experiment (in vivo experiment) for confirming efficacy of customized dermis regeneration sheet

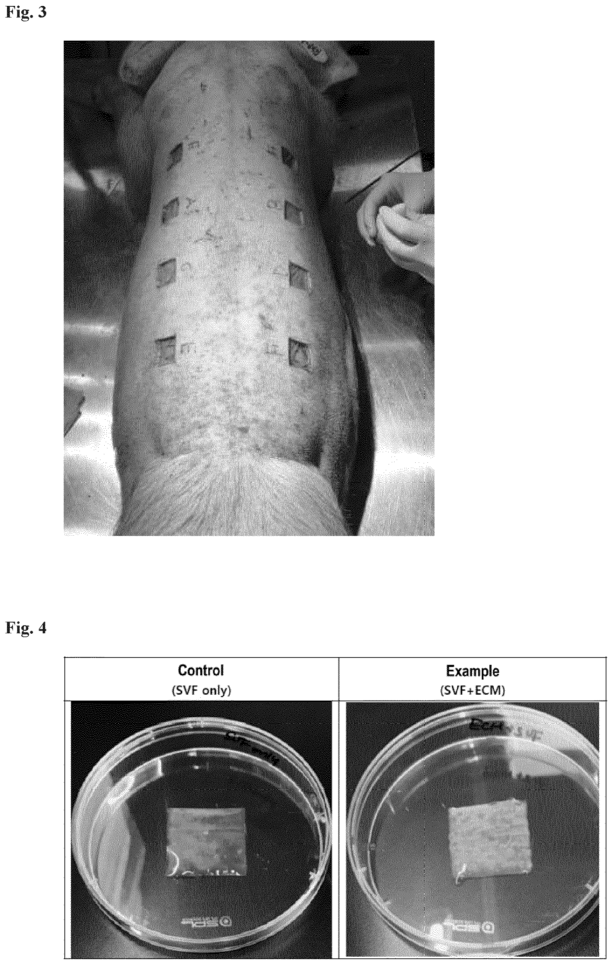

In order to verify the efficacy of the dermis regeneration sheet according to the present invention, a 2.5 cm × 2.5 cm piece of skin of a four-month-old pig was removed, and then SVF and ECM were obtained from an adipose tissue of the pig in the same manner as described above. FIG 3 shows a photograph in which the skin is removed in an animal experiment for Experimental Example 2.

Furthermore, after the obtained SVF and ECM were mixed using a mix syringe, a first liquid was prepared by mixing 1 ml of an aprotinin liquid in which 9 mg/ml fibrinogen was mixed with the SVF/ECM mixture using a mix syringe. In this case, the content of the ECM in the first liquid was 20 wt%, and the concentration of the SVF was 6.25 × 10⁶ cells/ml. Further, a second liquid comprising thrombin at a concentration of 50 lU/ml was prepared.

Moreover, after the removed skin region was scanned using a 3D scanner, the prepared first liquid and second liquid were sequentially applied using a 3D Bio 3D printer (INVIVO, ROKIT), and then a customized dermis regeneration sheet was manufactured by hardening the first liquid and the second liquid for 5 minutes. After the dermis regeneration sheet manufactured as described above was implanted into the removed skin region and subjected to dressing treatment, the degree of restoration of the skin was observed for 14 days.

As a control, a customized dermis regeneration sheet was manufactured in the same manner as described above without including the ECM in the first liquid and implanted into the removed skin region and subjected to dressing treatment, and then the degree of restoration of the skin was observed for 14 days.

FIG 4 shows a control prepared according to Experimental Example 2 and a customized dermis regeneration sheet of the Example.

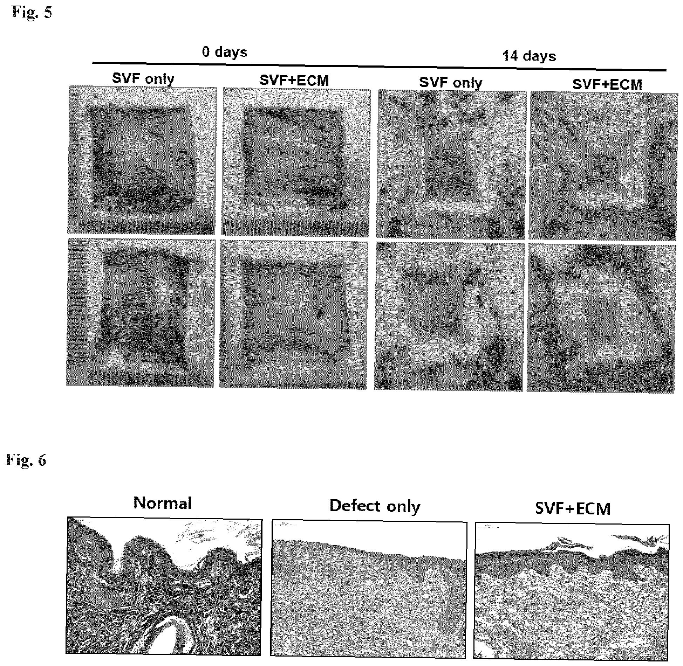

FIG 5 shows the progress of the implanted customized dermis regeneration sheets according to Experimental Example 2. Specifically, FIG 5 shows photographs in which the customized dermis regeneration sheets according to a control (only SVF) and an Example (SVF+ECM) are implanted, and shows the progress from the date of implantation (0 days) to 14 days after implantation.

FIG 6 shows the results of performing Masson's Trichrome staining in an animal experiment of Experimental Example 2. Specifically, in FIG 6, each of a non-damaged skin (normal) region, a skin-removed region (defect only), and a region (SVF+ECM) in which the customized dermis regeneration sheet according to the Example is implanted into a skin-removed region was stained with Masson's Trichrome and observed 14 days after the skin is removed. According to FIG 6, it can be confirmed that when the skin is removed, and then left to stand for 14 days (defect only), a collagen complex is formed at a very low concentration in the dermis. In contrast, it can be confirmed that when the customized dermis regeneration sheet according to the Example is implanted (SVF+ECM), the dermis is clearly stained blue, and thus a collagen complex is formed at high density in the dermis, and it can be confirmed that the dermis is clearly stained red, and thus keratin, which is a constituent component of the epidermis, cytoplasm, and the like are formed.

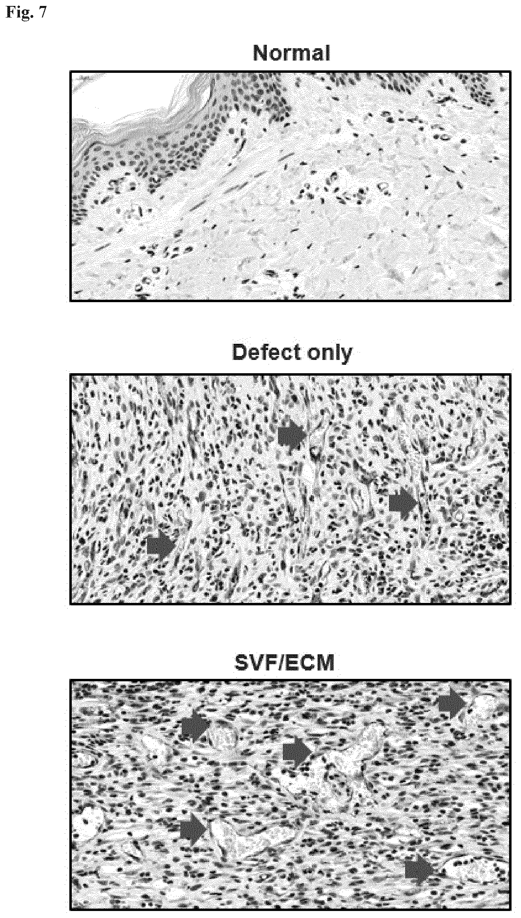

FIG 7 shows the presence or absence of angiogenesis at an implanted site as a result of the animal experiment according to Experimental Example 2 using CD31 staining. Specifically, in FIG 7, it is observed whether the blood vessels are regenerated by performing CD31 staining on each of a non-damaged skin (normal) region, a skin-removed region (defect only), and a region (SVF+ECM) in which the customized dermis regeneration sheet according to the Example is implanted into a skin-removed region 14 days after the skin is removed. According to FIG 7, it can be confirmed that when the skin is removed, and then left to stand for 14 days (defect only), angiogenic regions stained brown appear very sporadically and narrow. In contrast, it can be confirmed that when the customized dermis regeneration sheet according to the Example is implanted (SVF+ECM), there are a great number of angiogenic regions stained brown.

Based on various experimental results in Experimental Example 2, it can be seen that when the customized dermis regeneration sheet according to the present invention is implanted into the skin, the regeneration of the skin is promoted, and furthermore, angiogenesis in the skin is also promoted, so that the region in which the skin is removed may be restored similar to the original skin.

## Claims

1. A bioink composition for a dermis regeneration sheet, the bioink composition comprising:
a first liquid comprising an adipose tissue-derived stromal vascular fraction, an adipose tissue-derived extracellular matrix, and fibrinogen, wherein a content of the adipose tissue-derived extracellular matrix in the first liquid is 20 wt% to 60 wt%; and
a second liquid comprising thrombin, wherein a concentration of thrombin in the second liquid is 40 IU/ml to 250 IU/ml.

2. The bioink composition of claim 1, wherein a concentration of the adipose tissue-derived stromal vascular fraction in the first liquid is 10⁵ cells/ml to 10⁷ cells/ml.

3. The bioink composition of claim 1, wherein a content of the adipose tissue-derived extracellular matrix in the first liquid is 20 wt% to 50 wt%.

4. The bioink composition of claim 1, wherein the adipose tissue-derived extracellular matrix is particles having a diameter of 5 µm to 100µm.

5. The bioink composition of claim 1, wherein a concentration of fibrinogen in the first liquid is 4 mg/ml to 50 mg/ml.

6. The bioink composition of claim 1, wherein a concentration of thrombin in the second liquid is 40 IU/ml to 100 IU/ml.

7. The bioink composition of claim 1, wherein the adipose tissue-derived stromal vascular fraction and the adipose tissue-derived extracellular matrix are each extracted from an autologous adipose tissue.

8. The bioink composition of claim 1, wherein the first liquid further comprises aprotinin.

9. A method for manufacturing a customized dermis regeneration sheet, the method comprising:
a) obtaining 3D data of a defect dermis site using a 3D scanner;
a1) manufacturing a mold corresponding to a shape of the obtained 3D data using a 3D printer;
b) forming a first layer corresponding to a shape of the obtained 3D data using a first liquid comprising an adipose tissue-derived stromal vascular fraction, an adipose tissue-derived extracellular matrix, and fibrinogen, wherein a content of the adipose tissue-derived extracellular matrix in the first liquid is 20 wt% to 60 wt%, and wherein in step b), the first liquid is applied in the mold;
c) forming a second layer by applying a second liquid comprising thrombin onto the first layer, wherein a concentration of thrombin in the second liquid is 40 IU/ml to 250 IU/ml; and
d) forming a dermis regeneration sheet by allowing the first layer and the second layer to react with each other.

10. The method of claim 9, wherein steps b) and c) are alternately repeated.

11. The method of claim 9, wherein steps b) and c) are performed using inkjet printing or 3D printing.

12. A customized dermis regeneration sheet manufactured using the method of claim 9.

## Patentansprüche

1. Eine Biotintenzusammensetzung für ein Dermis-Regenerationsblatt, wobei die Biotintenzusammensetzung umfasst:
eine erste Flüssigkeit, die eine aus Fettgewebe stammende stromale vaskuläre Fraktion, eine aus Fettgewebe stammende extrazelluläre Matrix und Fibrinogen umfasst, wobei der Gehalt der aus Fettgewebe stammenden extrazellulären Matrix in der ersten Flüssigkeit 20 Gew.-% bis 60 Gew.-% beträgt; und
eine zweite Flüssigkeit, die Thrombin enthält, wobei die Konzentration von Thrombin in der zweiten Flüssigkeit 40 IU/ml bis 250 IU/ml beträgt.

2. Die Biotintenzusammensetzung gemäß Anspruch 1, wobei die Konzentration der aus Fettgewebe stammenden stromalen vaskulären Fraktion in der ersten Flüssigkeit 10⁵ Zellen/ml bis 10⁷ Zellen/ml beträgt.

3. Die Biotintenzusammensetzung gemäß Anspruch 1, wobei der Gehalt der aus Fettgewebe stammenden extrazellulären Matrix in der ersten Flüssigkeit 20 Gew.-% bis 50 Gew.-% beträgt.

4. Die Biotintenzusammensetzung gemäß Anspruch 1, wobei die aus Fettgewebe gewonnene extrazelluläre Matrix aus Partikeln mit einem Durchmesser von 5 µm bis 100µm besteht.

5. Die Biotintenzusammensetzung gemäß Anspruch 1, wobei die Konzentration von Fibrinogen in der ersten Flüssigkeit 4 mg/ml bis 50 mg/ml beträgt.

6. Die Biotintenzusammensetzung gemäß Anspruch 1, wobei die Thrombinkonzentration in der zweiten Flüssigkeit 40 IU/ml bis 100 IU/ml beträgt.

7. Die Biotintenzusammensetzung gemäß Anspruch 1, wobei die aus Fettgewebe stammende stromale vaskuläre Fraktion und die aus Fettgewebe stammende extrazelluläre Matrix jeweils aus autologem Fettgewebe gewonnen werden.

8. Die Biotintenzusammensetzung nach Anspruch 1, wobei die erste Flüssigkeit außerdem Aprotinin enthält.

9. Ein Verfahren zur Herstellung eines maßgeschneiderten Dermis-Regenerationsblattes, wobei das Verfahren umfasst:
a) Gewinnung von 3D-Daten einer Defektstelle in der Dermis mit einem 3D-Scanner;
a1) Herstellung einer Form, die einer Form der erhaltenen 3D-Daten entspricht, mit einem 3D-Drucker;
b) Bilden einer ersten Schicht, die einer Form der erhaltenen 3D-Daten entspricht, unter Verwendung einer ersten Flüssigkeit, die eine aus Fettgewebe stammende stromale vaskuläre Fraktion, eine aus Fettgewebe stammende extrazelluläre Matrix und Fibrinogen umfasst, wobei ein Gehalt der aus Fettgewebe stammenden extrazellulären Matrix in der ersten Flüssigkeit 20 Gew.-% bis 60 Gew.-% beträgt, und wobei in Schritt b) die erste Flüssigkeit in die Form eingebracht wird;
c) Bilden einer zweiten Schicht durch Auftragen einer zweiten Thrombin enthaltenden Flüssigkeit auf die erste Schicht, wobei die Konzentration von Thrombin in der zweiten Flüssigkeit 40 IE/ml bis 250 IE/ml beträgt; und
d) Bilden eines Dermis-Regenerationsblattes, indem man die erste Schicht und die zweite Schicht miteinander reagieren lässt.

10. Das Verfahren gemäß Anspruch 9, wobei die Schritte b) und c) abwechselnd wiederholt werden.

11. Das Verfahren gemäß Anspruch 9, wobei die Schritte b) und c) mittels Tintenstrahldruck oder 3D-Druck durchgeführt werden.

12. Ein maßgeschneidertes Dermis-Regenerationsblatt, hergestellt nach dem Verfahren von Anspruch 9.

## Revendications

1. Composition de bio-encre pour une feuille de régénération du derme, la composition de bio-encre comprenant :
un premier liquide comprenant une fraction vasculaire stromale dérivée de tissu adipeux, une matrice extracellulaire dérivée de tissu adipeux, et du fibrinogène, dans lequel la teneur en la matrice extracellulaire dérivée de tissu adipeux dans le premier liquide est de 20 % en poids à 60 % en poids ; et
un deuxième liquide comprenant du thrombine, dans lequel la concentration de thrombine dans le deuxième liquide est de 40 Ul/ml à 250 UI/ml.

2. Composition de bio-encre selon la revendication 1, dans laquelle la concentration de la fraction vasculaire stromale dérivée de tissu adipeux dans le premier liquide est de 10⁵ cellules/ml à 10⁷ cellules/ml.

3. Composition de bio-encre selon la revendication 1, dans laquelle la teneur en la matrice extracellulaire dérivée de tissu adipeux dans le premier liquide est de 20 % en poids à 50 % en poids.

4. Composition de bio-encre selon la revendication 1, dans laquelle la matrice extracellulaire dérivée de tissu adipeux consiste en particules ayant un diamètre de 5 µm à 100 µm.

5. Composition de bio-encre selon la revendication 1, dans laquelle la concentration de fibrinogène dans le premier liquide est de 4 mg/ml à 50 mg/ml.

6. Composition de bio-encre selon la revendication 1, dans laquelle la concentration de thrombine dans le deuxième liquide est de 40 Ul/ml à 100 Ul/ml.

7. Composition de bio-encre selon la revendication 1, dans laquelle la fraction vasculaire stromale dérivée de tissu adipeux et la matrice extracellulaire dérivée de tissu adipeux sont chacune extraites à partir d'un tissu adipeux autologue.

8. Composition de bio-encre selon la revendication 1, dans laquelle le premier liquide comprend en outre de l'aprotinine.

9. Procédé de fabrication d'une feuille de régénération du derme personnalisée, le procédé comprenant :
a) l'obtention de données 3D d'un site de défaut du derme utilisant un scanneur 3D ;
a1) la fabrication d'un moule correspondant à la forme des données 3D obtenues utilisant une imprimante 3D ;
b) la formation d'une première couche correspondant à la forme des données 3D obtenues utilisant un premier liquide comprenant une fraction vasculaire stromale dérivée de tissu adipeux, une matrice extracellulaire dérivée de tissu adipeux, et du fibrinogène, dans laquelle la teneur en la matrice extracellulaire dérivée de tissu adipeux dans le premier liquide est de 20 % en poids à 60 % en poids et dans laquelle, dans l'étape b), le premier liquide est appliqué dans le moule ;
c) la formation d'une deuxième couche par application d'un deuxième liquide comprenant de la thrombine sur la première couche, dans laquelle la concentration de thrombine dans le deuxième liquide est de 40 Ul/ml à 250 Ul/ml ;
d) la formation d'une feuille de régénération du derme par l'opération consistant à laisser la première couche et la deuxième couche réagir l'une avec l'autre.

10. Procédé selon la revendication 9, dans lequel les étapes b) et c) sont répétées en alternance.

11. Procédé selon la revendication 9, dans lequel les étapes b) et c) sont effectuées en utilisant une impression par jet d'encre ou une impression 3D.

12. Feuille de régénération du derme personnalisée fabriquée par utilisation du procédé de la revendication 9.
